(19) Europäisches Patentamt
European Patent Office
Office européen des brevets

(11) Numéro de publication: **0 036 357 B1**

# (12) FASCICULE DE BREVET EUROPEEN

(45) Date de publication du fascicule du brevet: **30.05.84**

(51) Int. Cl.$^3$: **C 07 D 253/06**, A 61 K 31/53, C 07 D 405/06

(21) Numéro de dépôt: **81400355.4**

(22) Date de dépôt: **09.03.81**

## (54) Dérivés cétoniques de diaryl 5-6 triazines 1-2-4 antalgiques et antiagrégants utilisables dans le traitement de fond des migraines.

(30) Priorité: **14.03.80 FR 8005859**

(43) Date de publication de la demande: **23.09.81 Bulletin 81/38**

(45) Mention de la délivrance du brevet: **30.05.84 Bulletin 84/22**

(84) Etats contractants désignés: **AT BE CH DE GB IT LI LU NL SE**

(56) Documents cités:
**FR - A - 2 368 278**
**FR - A - 2 383 176**
**FR - A - 2 417 508**

**Le dossier contient des informations techniques présentées postérieurement au dépôt de la demande et ne figurant pas dans le présent fascicule.**

(73) Titulaire: **PIERRE FABRE S.A., 125, rue de la Faisanderie, F-75116 Paris (FR)**

(72) Inventeur: **Pitet, Guy, 3, rue de l'Aubisque, F-31000 Toulouse (FR)**
Inventeur: **Couret, Françoise, Chemin de Thil, F-31450 Corransac (FR)**
Inventeur: **Cousse, Henri, La Foun de Los Nobios Chemin de Lastinos, F-81100 Castres (FR)**
Inventeur: **Mouzin, Gilbert, 21, rue Sainte Foy, F-81100 Castres (FR)**

(74) Mandataire: **Doat, Jean Pierre, 17, Avenue Jean Moulin, F-81106 Castres Cedex (FR)**

EP 0 036 357 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouveaux composés chimiques, des dérivés de diaryl 5-6 triazines 1-2-4, leur procédé de préparation et leur application.

Ces nouveaux principes actifs possèdent des propriétés antalgiques et antiagrégantes. Ils sont utiles notamment dans le traitement des migraines et la prévention des athéromes.

L'invention vise également les compositions pharmaceutiques les contenant.

Les composés chimiques objet de l'invention répondent à la formule générale:

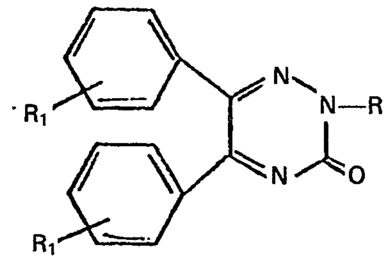

$R_1$ représente chaque fois un atome d'hydrogène, d'halogène, un groupe méthoxy ou diméthylamino.

R est un groupe contenant une fonction cétonique; ce groupe peut se schématiser:

$$-(CH_2)_n-CO-(CH_2)_n-H \qquad \text{ou}$$

$$-(CH_2)_n-CO-Ar$$

n représente les valeurs 1 à 5, Ar représente le groupe phényle, benzyle, naphtyle ou furyle pouvant être substitué par le brome ou par le groupe hydroxyle.

Dans les brevets français n° 7 632 162 et n° 7 707 245, déposés au nom de la demanderesse, il a été démontré que les composés triaziniques substitués en position 2 par des groupes alcoyls possédaient des propriétés antalgiques.

En poursuivant les investigations dans cet axe thérapeutique et sur ce type de structure, il a été obtenu de nouveaux composés chimiques possédant des propriétés antalgiques ayant un spectre d'activité plus large qui permet d'envisager compte tenu d'un fort pouvoir anti-agrégant l'utilisation en thérapeutique pour le traitement de fond des migraines, la prévention des athéromes et le traitement d'algies diverses.

A titre d'exemples non limitatifs, il est décrit ci-après quelques composés chimiques et leur mode de préparation.

*Schéma de synthèse*

Les diaryl triazines de formule générale:

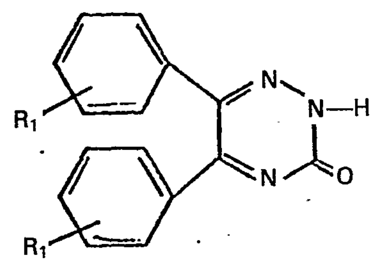

$R_1$ tels que définis précédemment sont obtenues par action du semicarbazide sur les diaryls dicétones correspondantes selon le schéma:

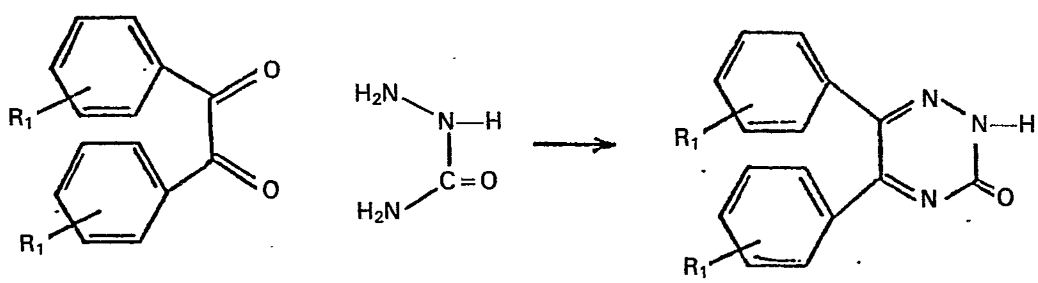

Les diaryls dicétones sont préparées selon des procédés décrits dans la litérature et plus particulièrement selon la mise au point à paraître dans Bollettino Chemico Farmaceutico 1980 de G. PITET, H. COUSSE et G. MOUZIN intitulée: les diaryls dicétones, synthèse et utilisation pour l'obtention de médicaments et spécialement des as triazines.

A partir des composés précédents, l'introduction du substituant R contenant la fonction cétonique peut être réalisée selon deux modes opératoires de synthèse:

*Procédé 1* (mode opératoire A)

Une mole de diaryl triazine en suspension dans le DMF est traitée par une mole d'hydrure de sodium puis par une quantité stoechiométrique de réactif halogéné:

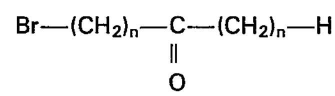

*Procédé 2* (mode opératoire B)

Une mole de triazine est traitée par 2 litres de soude à 50% dans l'eau, ajouter 2 litres de chlorure de méthylène et 3,5 g de chlorure de benzyl triéthyl ammonium. Ce milieu réactionnel est ensuite traité par le composé halogéno cétonique.

En opérant ainsi, il a été obtenu les composés selon la formule générale. Selon le mode opératoire A, il a été synthétisé les composés (Me = $CH_3$).

*Exemple 1*

Oxo-3 acétonyl-2 di(para-méthoxy phényl) 5-6 as triazine

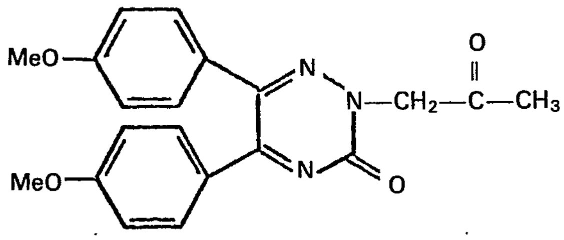

Cristaux jaunes.
Soluble dans l'éthanol, le benzène, le chlorure de méthylène et le chloroforme.
Insoluble dans l'eau, les acides et bases dilués, l'éther.
Point de fusion: 145°C.

*Exemple 2*

Oxo-3 α méthylacétonyl-2 di(para-méthoxyphényl) 5-6 as triazine

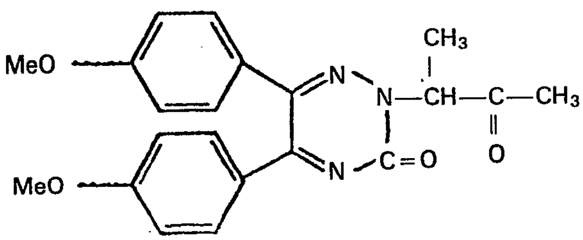

Cristaux jaunes.
Soluble dans l'éthanol, le benzène, le chlorure de méthylène et le chloroforme.
Insoluble dans l'eau, les acides et bases diluées, et l'éther.
Point de fusion: 114°C.

*Exemple 3*

Oxo-3 α méthylacétonyl-2 di(para-diméthyl amino phényl) 5-6 as triazine

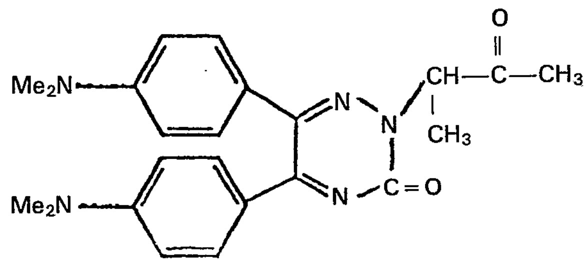

Cristaux oranges.
Soluble dans les acides dilués aqueux, l'éthanol, le benzène, le chlorure de méthylène et le chloroforme.
Insoluble dans l'eau, les bases diluées et l'éther.
Point de fusion: 194°C.

*Exemple 4*

Oxo-3 acétophényl-2 di(para-diméthylaminophényl) 5-6 as triazine

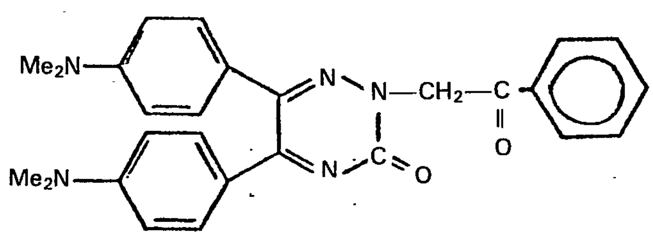

Cristaux oranges.
Soluble dans les acides dilués aqueux, le chlorure de méthylène et le chloroforme.
Insoluble dans l'eau, les bases diluées, le benzène, l'éthanol et l'éther.

Point de fusion: 258°C.

*Exemple 5*

Oxo-3 (3,4 dihydroxyphénacyl)-2 di(para-méthoxy phényl) 5-6 as triazine

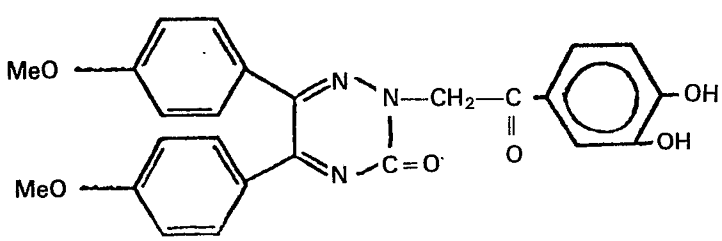

Cristaux jaunes.
Soluble dans les bases diluées et l'éthanol.
Insoluble dans l'eau, les acides dilués, le benzène, le chlorure de méthylène et l'éther.
Point de fusion: 192°C puis 224°C.

*Exemple 6*

Oxo-3 (3,4 dihydroxyphénacyl)-2 di(para-diméthyl-aminophényl) 5-6 as triazine

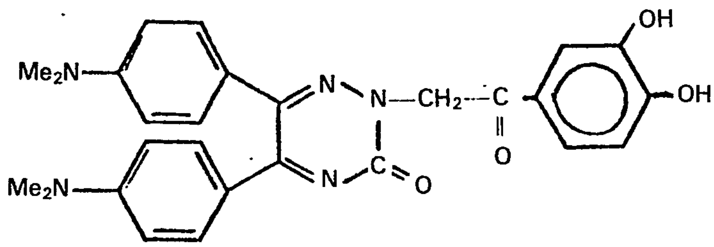

Cristaux rouges.
Soluble dans les acides et les bases dilués et l'éthanol.
Insoluble dans l'eau, le benzène, le chlorure de méthylène, l'éther.

Point de fusion: 260°C.

*Exemple 7*

Oxo-3 (para-bromophénacyl)-2 di(para-méthoxyphényl) 5-6 as triazine

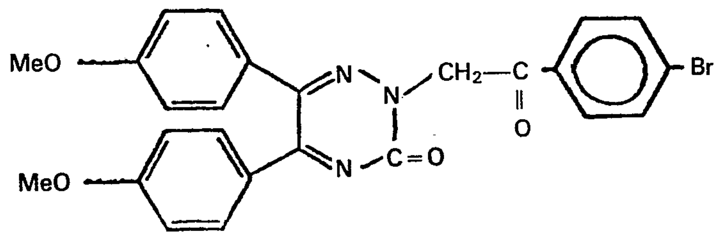

Cristaux jaunes.
Soluble dans l'éthanol, le benzène, le chlorure de méthylène, le chloroforme.
Insoluble dans l'eau, les acides et bases dilués.
Point de fusion: 200°C.

*Exemple 8*

Oxo-3 acétonyl-2 di(para-diméthylaminophényl) 5-6 as triazine

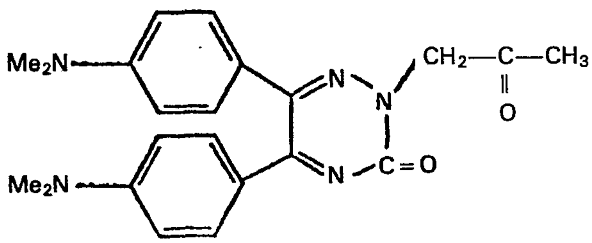

Cristaux oranges.
Insoluble dans l'eau et les bases diluées, le benzène et l'éther.
Soluble dans les acides dilués, l'éthanol, le chlorure de méthylène et le chloroforme.

Point de fusion: 228°C.

*Exemple 9*

Oxo-3 ( $\delta$ propylméthylcétone)-2 di(para-diméthylaminophényl) 5-6 as triazine

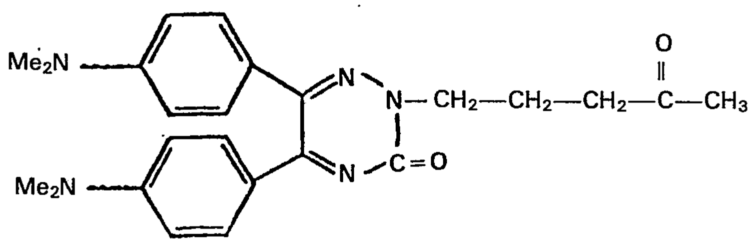

Nous avons synthétisé selon le mode opératoire B les composés suivantes.

*Exemple 10*

Oxo-3 ( $\gamma$ diéthylcétone)-2 di(para-diméthylaminophényl) 5-6 as triazine

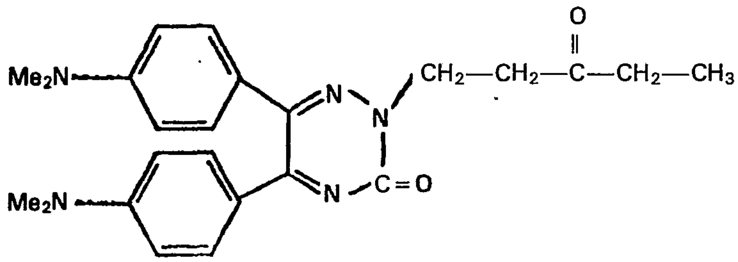

Cristaux jaune-oranges.
Insoluble dans l'eau, les bases diluées et l'éther.
Soluble dans les acides dilués, l'éthanol, le benzène, le chlorure de méthylène et le chloroforme.
Point de fusion: 175°C.

*Exemple 11*

Oxo-3 ( $\gamma$ diéthylcétone)-2 di(para-méthoxyphényl) 5-6 as triazine

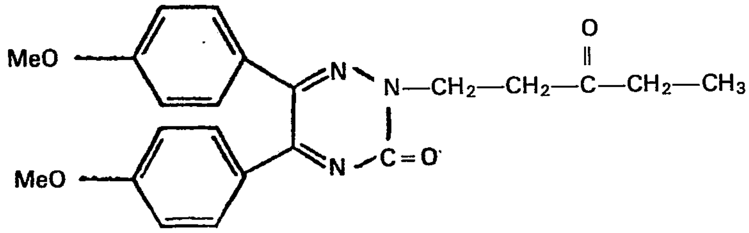

Cristaux jaunes.
Insoluble dans l'eau, les bases et les acides dilués et l'éther.
Soluble dans l'éthanol, le benzène, le chlorure de méthylène et le chloroforme.
Point de fusion: 100°C.

*Experimentations*

Les composés chimiques précédemment décrits ont fait l'objet d'essais toxicologiques, pharmacologiques et cliniques qui ont permis de mettre en évidence d'intéressantes propriétés antalgiques.

*a) Toxicologie*

L'étude de la toxicité a été effectuée chez la souris conventionnelle pesant environ 20 grammes.

Les substances ont été administrées par voie orale et intrapéritonéale. Les doses létales 50 sont exprimées en mg/kg et ont été calculées selon la méthode de MILLER et TAINTER, Proc. Soc. Exper. Biol. Med. 1944, 57, 261.

A titre d'exemple dans le tableau ci-dessous figurent quelques valeurs de doses toxiques provoquant 50% de mortalité.

| Composés/ Exemple | $DL_{50}$ voie orale mg/kg | $DL_{50}$ voie I.P. mg/kg |
|---|---|---|
| 1 | > 1500 | > 500 |
| 2 | > 2000 | > 1000 |
| 3 | > 1500 | |
| 4 | > 1000 | ≃ 750 |
| 5 | > 1000 | |
| 6 | > 1000 | |
| 7 | > 2000 | ≃ 800 |
| 8 | > 1500 | |
| 9 | > 1500 | |
| 10 | > 1000 | > 500 |
| 11 | > 2000 | |

Aux doses administrées, les doses létales 50 n'ont pas été atteintes.

Compte-tenu de leur activité, ces composés chimiques présentent des index thérapeutiquement très élevés, plus particulièrement ceux des exemples 1, 2, 3 et 8.

*b) Propriétés antalgiques*

L'activité sur le test des contorsions à la phényl benzoquinone (selon SIEGMUND et Coll. - J. Pharm. Exptl. Ther. 1957, 119, 453) a été déterminée après administration du produit per os; la dose est exprimée en mg/kg chez la souris 30 minutes avant l'injection intra-péritonéale de l'agent algogène.

Les résultats sont exprimés en pourcentage de variation du nombre de contorsions; pour les composés les plus actives, nous avons déterminé la dose qui provoque 50% de diminution des contorsions $DE_{50}$.

Dans le tableau ci-dessous nous avons répertorié les composés dont l'activité est supérieure ou égale à celle de la glafénine ($DE_{50} \simeq 40$).

| Composé/ Exemple | % diminution à 100 mg/kg | $DE_{50}$ mg/kg |
|---|---|---|
| 1 | — 80% | 15 |
| 2 | — 90% | 10 |
| 3 | — 90% | 5 |
| 8 | — 93% | 4 |
| 9 | — 83% | 7 |
| 10 | — 80% | 5 |

*c) Applications*

Ces composés et plus particulièrement ceux des exemples 1 et 2 qui sont les moins colorants et les mieux tolérés par administration répétée sont utilisables dans le traitement des algies et plus particulièrement pour le traitement de fond des migraines.

Ces composés sont dépourvus d'effet anti-inflammatoire et nous n'avons constaté aucun effet ulcérogène selon les critères de PFEIFER et LEWAL DOWFKI (Arch. Int. Pharmacodyn. 1971, *190,* 5).

**Revendications**

1. Les composés chimiques de formule générale

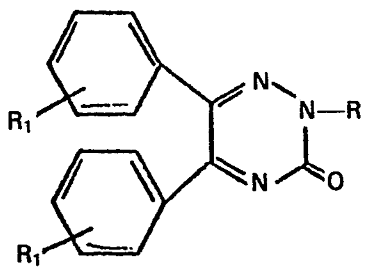

dans laquelle $R_1$ représente chaque fois un atome d'hydrogène, d'halogène, un groupe méthoxy ou diméthylamino.

R représente un groupe possédant une fonction cétonique; ce groupe peut se schématiser:

$$—(CH_2)_n—\overset{\overset{O}{\|}}{C}—(CH_2)_n—H$$ ou

$$(CH_2)_n—\overset{\overset{O}{\|}}{C}—Ar$$

n représente les valeurs 1 à 5, Ar représente le groupe phényle, benzyle, naphtyle ou furyle, pouvant être substitué par le brome ou par le groupe hydroxyle.

2. Oxo-3 acétonyl-2 di(para-méthoxyphényl) 5-6 as triazine.

3. Oxo-3 α méthylacétonyl-2 di(para-méthoxyphényl) 5-6 as triazine.

4. Oxo-3 α méthylacétonyl-2 di(para-diméthylaminophényl) 5-6 as triazine.

5. Oxo-3 acétophényl-2 di(para-diméthylaminophényl) 5-6 as triazine.

6. Oxo-3 (3,4 dihydroxyphénacyl)-2 di(para-méthoxyphényl) 5-6 as triazine.

7. Oxo-3 (3,4 dihydroxyphénacyl)-2 di(para-diméthylaminophényl) 5-6 as triazine.

8. Oxo-3 (para-bromophénacyl)-2 di(para-méthoxyphényl) 5-6 as triazine.

9. Oxo-3 acétonyl-2 di(para-diméthylaminophényl) 5-6 as triazine.

10. Oxo-3 (δ propylméthylcétone)-2 di(para-diméthylaminophényl) 5-6 as triazine.

11. Oxo-3 (γ diéthylcétone)-2 di(para-diméthylaminophényl) 5-6 as triazine.

12. Oxo-3 (γ diéthylcétone)-2 di(para-méthoxyphényl) 5-6 as triazine.

13. Un procédé de préparation des composés selon la formule générale de la revendication 1, caractérisé en ce que l'on traite une diaryl triazine, correspondante, en suspension dans le diméthyl formamide, par l'hydrure de sodium, puis que l'on condense avec une halogénocétone correspondante.

14. Un procédé de préparation des composés selon la formule générale de la revendication 1 et plus particulièrement les dérivés des revendications 11 et 12, caractérisé en ce que l'on traite une diaryl triazine correspondante avec une solution aqueuse à 50% de soude en présence du chlorure de benzyl triéthylammonium, puis que l'on condense avec une halogénocétone correspondante.

15. A titre de médicaments utilisables en thérapeutique humaine ou vétérinaire les composés selon les revendications 1 à 12 doués de propriétés antalgiques et antiagrégantes.

16. Les compositions contenant à titre de principe actif un composé selon les revendications 1 à 12 et 15 utilisables en thérapeutique et plus particulièrement dans le traitement des migraines.

17. Les compositions selon la revendication 16 contenant d'autres principes actifs associés aux dérivés des revendications 1 à 12.

18. Les compositions selon les revendications 16 et 17 contenant les excipients appropriés et les véhicules galéniques permettant l'administration par voie orale, injectable, locale ou rectale.

**Claims**

1. The chemical compounds of the general formula

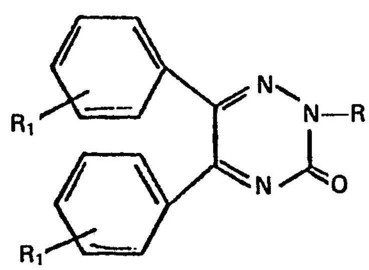

in which $R_1$ represents, each time, a hydrogen atom, a halogen atom, a methoxy group or a dimethylamino group, R represents a group having a ketonic function; this group may be shown schematically:

$$—(CH_2)_n—\overset{\overset{O}{\|}}{C}—(CH_2)_n—H$$ or

$$(CH_2)_n—\overset{\overset{O}{\|}}{C}—Ar$$

n represents the values 1 to 5, and
Ar represents a phenyl group, a benzyl group, a naphthyl group or a furyl group, each of which may be substituted by bromine or by the hydroxy group.

2. 3-oxo-2-acetonyl-5,6-di(para-methoxyphenyl)-asym-triazine.

3. 3-oxo-2-(1-methyl-2-oxopropyl)-5,6-di-(para-methoxyphenyl)-asym-triazine.

4. 3-oxo-2-(1-methyl-2-oxopropyl)-5,6-di-(para-dimethylaminophenyl)-asym-triazine.

5. 3-oxo-2-phenacyl-5,6-di(para-dimethylaminophenyl)-asym-triazine.

6. 3-oxo-2-(3,4-dihydroxyphenacyl)-5,6-di(para-methoxyphenyl)-asym-triazine.

7. 3-oxo-2-(3,4-dihydroxyphenacyl)-5,6-di(para-dimethylaminophenyl)-asym-triazine.

8. 3-oxo-2-(para-bromophenacyl)-5,6-di(para--methoxyphenyl)-asym-triazine.

9. 3-oxo-2-acetonyl-5,6-di(para-dimethylaminophenyl)-asym-triazine.

10. 3-oxo-2-(4-oxopentyl)-5,6-di-(para-dimethylaminophenyl)-asym-triazine.

11. 3-oxo-2-(3-oxopentyl)-5,6-di-(para-dimethylaminophenyl)-asym-triazine.

12. 3-oxo-2-(3-oxopentyl)-5,6-di-(para-methoxyphenyl)-asym-triazine.

13. A process for the preparation of the compounds according to the general formula of claim 1, characterised in that a corresponding diaryltriazine in suspension in dimethylformamide is treated with sodium hydride and then condensed with a corresponding haloketone.

14. A process for the preparation of the compounds according to the general formula of claim 1, and more especially the derivatives of claims 11 and 12, characterised in that a corresponding diaryltriazine is treated with a 50% aqueous sodium hydroxide solution in the presence of benzyltriethylammonium chloride and then condensed with a corresponding haloketone.

15. The compounds according to claims 1 to 12 having analgesic and anti-aggregant properties as medicaments that can be used for human or veterinary therapy.

16. Compositions containing as active ingredient a compound according to claims 1 to 12 and 15 that can be used in therapy and, more especially, in the treatment of migraines.

17. Compositions according to claim 16 containing other active ingredients in conjunction with the derivatives of claims 1 to 12.

18. Compositions according to claims 16 and 17 containing suitable excipients and galenic carriers permitting oral, rectal or local administration or administration by injection.

**Patentansprüche**

1. Chemische Verbindungen mit der allgemeinen Formel

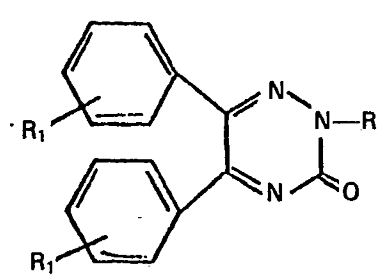

worin $R_1$ jeweils ein Wasserstoffatom, Halogen, eine Methoxygruppe oder Dimethylaminogruppe bedeutet. R bedeutet eine Gruppe enthaltend eine Ketonfunktion; diese Gruppe kann dargestellt werden:

$$-(CH_2)_n-\overset{\overset{\displaystyle O}{\|}}{C}-(CH_2)_n-H$$

oder

$$(CH_2)_n-\overset{\overset{\displaystyle O}{\|}}{C}-Ar$$

n bedeutet einen Wert von 1 bis 5. Ar bedeutet eine Phenyl-, Benzyl-, Naphthyl- oder Furylgruppe, die durch Brom oder eine Hydroxylgruppe substituiert sein kann.

2. Oxo-3-acetonyl-2-di-(para-methoxyphenyl)--5,6-as-triazin.

3. Oxo-3-α-methylacetonyl-2-di-(para-methoxyphenyl)-5,6-as-triazin.

4. Oxo-3-α-methylacetonyl-2-di-(para-dimethylaminophenyl)-5,6-as-triazin.

5. Oxo-3-acetophenyl-2-di-(para-dimethylaminophenyl)-5,6-as-triazin.

6. Oxo-3-(3,4-dihydroxyphenacyl)-2-di-(para-methoxyphenyl)-5,6-as-triazin.

7. Oxo-3-(3,4-dihydroxyphenacyl)-2-di-(para-dimethylaminophenyl)-5,6-as-triazin.

8. Oxo-3-(para-bromphenacyl)-2-di-(para-methoxyphenyl)-5,6-as-triazin.

9. Oxo-3-acetonyl-2-di-(para-dimethylaminophenyl)-5,6-as-triazin.

10. Oxo-3-(δ-propylmethylketon)-2-di-(para-dimethylaminophenyl)-5,6-as-triazin.

11. Oxo-3-(γ-diethylketon)-2-di-(para-dimethylaminophenyl)-5,6-as-triazin.

12. Oxo-3-(γ-diethylketon)-2-di-(para-methoxyphenyl)-5,6-as-triazin.

13. Verfahren zur Herstellung der Verbindungen gemäss der allgemeinen Formel von Anspruch 1, dadurch gekennzeichnet, dass man ein entsprechendes Diaryltriazin, gelöst in Dimethylformamid, behandelt mit Natriumhydrid und dann kondensiert mit einem entsprechenden Halogenketon.

14. Verfahren zur Herstellung der Verbindungen gemäss der allgemeinen Formel von Anspruch 1 und insbesondere der Derivate der Ansprüche 11 und 12, dadurch gekennzeichnet, dass man ein entsprechendes Diaryltriazin behandelt mit einer wässrigen Lösung von 50%igem Natriumcarbonat in Anwesenheit von Triethylammoniumbenzylchlorid und dann mit einem entsprechenden Halogenketon kondensiert.

15. Als Medikamente in der Human- oder Veterinärtherapie brauchbare Verbindungen gemäss den Ansprüchen 1 bis 12 mit schmerzstillenden und antiagregierenden Eigenschaften.

16. Zusammensetzungen enthaltend als aktiven Bestandteil eine Verbindung nach einem der Ansprüche 1 bis 12 und 15, brauchbar in der Therapie und insbesondere in der Behandlung der Migräne.

17. Zusammensetzungen nach Anspruch 16, enthaltend andere aktive Bestandteile zusammen mit Derivaten der Ansprüche 1 bis 12.

18. Zusammensetzungen nach den Ansprüchen 16 und 17, enthaltend geeignete Grundmassen und galenische Träger, die eine Verabreichung auf oralem Weg, als Injektion und lokal oder rektal ermöglichen.